# EUROPEAN PATENT APPLICATION

(11) **EP 4 524 256 A1**
(43) Date of publication of application: **19.03.2025**
(21) Application number: 22951725.5
(22) Date of filing: 19.09.2022
(51) Int. Cl.: C12Q 1/6806, C12Q 1/6811, C12Q 1/6876

(54) **BISPECIFIC-PROBE-COUPLED CAPTURE MAGNETIC BEAD, AND PREPARATION METHOD THEREFOR AND USE THEREOF**

(30) Priority: 20.07.2022 CN 202210861620
(71) Applicant: Sansure Biotech Inc., Changsha, Hunan 410205 (CN)
(72) Inventor: DAI, Lizhong, Changsha, Hunan 410205 (CN); ZHOU, Rong, Changsha, Hunan 410205 (CN); JI, Bozhi, Changsha, Hunan 410205 (CN); LIU, Jia, Changsha, Hunan 410205 (CN); GUO, Jun, Changsha, Hunan 410205 (CN)
(74) Representative: RGTH
(86) International application number: PCT/CN2022/119600
(87) International publication number: WO 2024/016461

(57) **Abstract**

Provided in the present application is a method for preparing a bispecific-probe-coupled capture magnetic bead, the method includes: coupling a capture magnetic bead to a bispecific probe, wherein hypoxanthine is introduced into a position in the 15-25 bp region at the 5' end of the bispecific probe, and the bispecific probe has a length of 20-70 bp.

## Description

### CROSS-REFERENCE TO RELATED APPLICATIONS

This application claims the priority of application CN202210861620.9, filed on July 20, 2022 and entitled "Bispecific-probe-coupled Capture Magnetic Bead, and Preparation Method Therefor and Use Thereof", which are incorporated herein by reference in its entirety.

### TECHNICAL FIELD

The application belongs to the field of biological detection, particularly relates to a method for preparing a capture magnetic bead, more particularly relates to a method for preparing a bispecific-probe-coupled capture magnetic bead.

### BACKGROUND

At present, screening technology based on DNA of complex samples (such as fecal samples) is used as a means of intestinal microbial testing or cancer screening due to its advantages of non-invasiveness and high sensitivity. A number of products for DNA-based intestinal cancer screening have been approved by the National Medical Products Administration, and their detection targets mainly include Septin9, SDC2, BMP3, NDRG4, and miRNA, etc.

Compared with early cancer screening methods based on detecting free DNA in plasma, urine, or hydrothorax and ascites of a patient, detecting fecal samples of a patient has higher sensitivity and accuracy. However, fecal samples include complex components, and most of them are various types of bacteria and food residues, which cause great background interference and inhibition to target detection. In addition, the content of cancer cells is low, thereby making the target detection rate low and unstable. At present, the common magnetic bead extraction kits on the market can meet the subsequent testing needs for the extraction and purification of samples such as plasma, urine, or hydrothorax and ascites. However, for complex samples such as fecal samples, it is difficult to enrich, extract, and purify the corresponding target fragments.

Based on this, some studies have combined DNA/RNA capture probes with the carboxyl groups of carboxyl magnetic beads to form capture magnetic beads, which can capture specific sequences in complex samples. However, the conventional magnetic bead capture probe currently used is a specific nucleotide sequence with a length of about 30-50 bp; the longer the length, the higher the capture specificity. However, a too-long sequence will cause itself to form a folded dimer or hairpin structure, affecting the subsequent magnetic bead capture efficiency. Especially when detecting methylated and unmethylated targets similar to colorectal cancer, the specific detection site is mainly the difference in CpG sites. Due to the particularity of this site, it is possible that the methylated and unmethylated targets differ by only one base to three bases during the detection process. Common capture probes can still bind and capture normally when a single base or even three bases are mismatched. This non-specific capture will lead to insufficient capture of nucleic acid or inaccurate detection of the target fragment.

Therefore, there is a need in the art for a method for specifically extracting target nucleic acids from complex samples with high accuracy and good sensitivity.

### SUMMARY

In view of this, in a first aspect, the present application provides a method for preparing a bispecific-probe-coupled capture magnetic bead, where the method includes the following steps:
coupling a capture magnetic bead with a bispecific probe,
where 5' end of the bispecific probe is modified with a primary amino group, and hypoxanthine is introduced into a position in the 15-25 bp region at the 5' end of the bispecific probe, and
the bispecific probe has a length of 20-70 bp.

In some particular embodiments, the number of hypoxanthine is 1-8.

In some particular embodiments, the number of hypoxanthine is 3-6.

In some particular embodiments, the number of hypoxanthine can be 1, 2, 3, 4, 5, 6, 7, or 8.

In some particular embodiments, the capture magnetic bead is a carboxyl magnetic bead, and the 5' end of the bispecific probe is modified with a primary amino group.

In some particular embodiments, the carboxyl groups of the carboxyl magnetic bead are activated, including the following steps:
S1. placing the carboxyl magnetic bead on a magnetic grate for adsorption;
S2. adding 2-morpholinoethanesulfonic acid to shake and mix well, then placing the mixture on a magnetic grate for adsorption.

More preferably, the carboxyl groups of the carboxyl magnetic beads are activated by repeating step S2.

In some particular embodiments, coupling a carboxyl magnetic beads with a bispecific probe includes the following steps:
mixing the bispecific probe with the activated carboxyl magnetic bead, 2-morpholinoethanesulfonic acid, and 1-(3-dimethylaminopropyl)-3-ethylcarbodiimide hydrochloride together to incubate with shaking.

In some particular embodiments, the preparation method further includes the following steps: blocking of the bispecific-probe-coupled capture magnetic bead.

In some particular embodiments, the blocking of the bispecific-probe-coupled capture magnetic bead includes the following steps: placing the bispecific-probe-coupled capture magnetic bead on a magnetic grate for adsorption after completion of the coupling, then adding PBST solution (phosphate-tween buffer) containing 1% BSA (bovine serum albumin), shaking to mix well for blocking.

In some particular embodiments, the target of the bispecific probe is SDC2 gene, NDRG4 gene, Septin9 gene, β-actin gene and/or *Escherichia coli* (*E. coli*)*.*

In some particular embodiments, the target of the bispecific probe is the SDC2 gene.

Furthermore, the probe sequence is:
5'-CGGCGTTTATTGGTTTTCGGAGTTGIIIITCGGCGTGTAA-3' (SEQ ID NO: 1).

In some particular embodiments, the target of the bispecific probe is the NDRG4 gene.

Furthermore, the probe sequence is:
5'-TTTATCGGGTATTTTAGTCIIIIAGAAGGCGGAAGTTACG-3' (SEQ ID NO: 2).

In some particular embodiments, the target of the bispecific probe is the Septin 9 gene.

Furthermore, the probe sequence is:
5'-TTAGTTATTATGTCGGATTTIIIIGTTAACGCGTAGTTGG-3' (SEQ ID NO: 3).

In some particular embodiments, the target of the bispecific probe is the β-actin gene.

Furthermore, the probe sequence is:
5'-TTGTAATTTTTAAGGGAGGAGIIIITTTTATTGGTT-3' (SEQ ID NO: 4).

Furthermore, the probe can also target *Escherichia coli,* and the sequence is
5'-AGTTTATCTGCAAGGTGAIIIITTAATTCCTCTCTTTCCT-3' (SEQ ID NO: 5).

The bispecific-probe-coupled capture magnetic bead obtained by the method for preparing magnetic beads according to the present application may better capture the target fragments, thereby ensuring the stability of the capture, and having higher sensitivity. For low-concentration nucleic acids in complex sample types, the detection rate may be significantly improved, and multi-step sample purification is not required, which reduces the number of operation steps and allows for rapid and efficient extraction of target nucleic acids. At the same time, it can effectively reduce erroneous capture caused by mismatches.

In a second aspect, the present application provides a bispecific-probe-coupled capture magnetic bead, where it is prepared by the above-mentioned preparation method.

In some particular embodiments, the target of the bispecific probe is SDC2 gene, NDRG4 gene, Septin9 gene, β-actin gene and/or *Escherichia coli.*

In some particular embodiments, the target of the bispecific probe is the SDC2 gene.

Furthermore, the probe sequence is:
5'-CGGCGTTTATTGGTTTTCGGAGTTGIIIITCGGCGTGTAA-3' (SEQ ID NO: 1).

In some particular embodiments, the target of the bispecific probe is the NDRG4 gene.

Furthermore, the probe sequence is:
5'-TTTATCGGGTATTTTAGTCIIIIAGAAGGCGGAAGTTACG-3' (SEQ ID NO: 2).

In some particular embodiments, the target of the bispecific probe is the Septin 9 gene.

Furthermore, the probe sequence is:
5'-TTAGTTATTATGTCGGATTTIIIIGTTAACGCGTAGTTGG-3' (SEQ ID NO: 3).

In some particular embodiments, the target of the bispecific probe is the β-actin gene.

Furthermore, the probe sequence is:
5'-TTGTAATTTTTAAGGGAGGAGIIIITTTTATTGGTT-3' (SEQ ID NO: 4).

Furthermore, the probe can also target *Escherichia coli,* the sequence is
5'-AGTTTATCTGCAAGGTGAIIIITTAATTCCTCTCTTTCCT-3' (SEQ ID NO: 5).

In a third aspect, the present application provides a kit for extracting nucleic acids, where it includes the bispecific-probe-coupled capture magnetic bead as described above.

Furthermore, the present application provides a kit for extracting nucleic acids from fecal samples, where it includes the bispecific-probe-coupled capture magnetic bead as described above.

In some particular embodiments, the kit includes polyethylene glycol.

Preferably, the concentration of the polyethylene glycol is 15%-35%.

More preferably, the concentration of the polyethylene glycol is 20%-30%.

Most preferably, the concentration of polyethylene glycol is 20%.

Using polyethylene glycol at an appropriate concentration can increase the capture efficiency of the bispecific-probe-coupled capture magnetic bead, and further improve the capture sensitivity of the bispecific-probe-coupled capture magnetic bead.

In some particular embodiments, the kit further includes a positive control and a negative control.

In a fourth aspect, the present application provides use of the bispecific-probe-coupled capture magnetic beadas described above in the preparation of a kit for extracting nucleic acids.

In a fifth aspect, the present application provides a method for extracting nucleic acids, which includes extracting a sample by using the bispecific-probe-coupled capture magnetic bead as described above.

Furthermore, the sample is feces, urine, plasma, or hydrothorax and ascites.

Preferably, the sample is feces.

### BRIEF DESCRIPTION OF THE DRAWINGS

Fig. 1 is a comparison of the effects of different types of modified magnetic beads on extracting nucleic acid of SDC2 gene from high-concentration fecal samples;
Fig. 2 is a comparison of the effects of different types of modified magnetic beads on extracting nucleic acid of SDC2 gene from low-concentration fecal samples;
Fig. 3 is a comparison of the effects of different types of modified magnetic beads on extracting nucleic acid of β-actin gene from high-concentration fecal samples;
Fig. 4 is a comparison of the effects of different types of modified magnetic beads on extracting nucleic acid of β-actin gene from low-concentration fecal samples;
Fig. 5 is a comparison of the effects of different types of modified magnetic beads on extracting nucleic acid of SDC2 gene from high-concentration plasma samples;
Fig. 6 is a comparison of the effects of different types of modified magnetic beads on extracting nucleic acid of SDC2 gene from low-concentration plasma samples;
Fig. 7 is a comparison of the effects of different types of modified magnetic beads on extracting nucleic acid of β-actin gene from high-concentration plasma samples;
Fig. 8 is a comparison of the effects of different types of modified magnetic beads on extracting nucleic acid of β-actin gene from low-concentration plasma samples;
Fig. 9 is a comparison of the effects of different types of modified magnetic beads on extracting nucleic acid of SDC2 gene from high-concentration urine samples;
Fig. 10 is a comparison of the effects of different types of modified magnetic beads on extracting nucleic acid of SDC2 gene from low-concentration urine samples;
Fig. 11 is a comparison of the effects of different types of modified magnetic beads on extracting nucleic acid of β-actin gene from high-concentration urine samples;
Fig. 12 is a comparison of the effects of different types of modified magnetic beads on extracting nucleic acid of β-actin gene from low-concentration urine samples;
Fig. 13 is a comparison of the effects of different types of modified magnetic beads on extracting nucleic acid of SDC2 gene from high-concentration hydrothorax and ascites samples;
Fig. 14 is a comparison of the effects of different types of modified magnetic beads on extracting nucleic acid of SDC2 gene from low-concentration hydrothorax and ascites samples;
Fig. 15 is a comparison of the effects of different types of modified magnetic beads on extracting nucleic acid of β-actin gene from high-concentration hydrothorax and ascites samples;
Fig. 16 is a comparison of the effects of different types of modified magnetic beads on extracting nucleic acid of β-actin gene from low-concentration hydrothorax and ascites samples;
Fig. 17 shows the extraction effects by using different concentrations of PEG;
Fig. 18 shows the effect of extracting nucleic acid of SDC2 gene by capture probes introduced hypoxanthine at different positions;
Fig. 19 shows the effect of extracting nucleic acid of NDRG4 gene by capture probes introduced hypoxanthine at different positions;
Fig. 20 shows the effect of extracting nucleic acid of Septin9 gene by capture probes introduced hypoxanthine at different positions;
Fig. 21 shows the effect of extracting nucleic acid of Septin9 introducing one mismatched base by different capture probes;
Fig. 22 shows the effect of extracting nucleic acid of Septin9 introducing three mismatched bases by different capture probes.

### DETAILED DESCRIPTION OF THE EMBODIMENTS

The present application will be described in detail below in conjunction with particular embodiments and examples, and the advantages and various effects of the present application will be more clearly presented. Those skilled in the art should understand that, these particular embodiments and examples are used to illustrate the present application rather than to limit the present application.

### Example 1: Preparation of Bispecific-probe-coupled Capture Magnetic Beads

### 1.1 Activating carboxyl groups:

1) 1 mL of carboxyl magnetic beads was taken out from refrigerator at 4°C to place on a magnetic grate for adsorption for 2 minutes, when the solution becomes clear, discarding the supernatant.
2) 1 mL of 2-morpholinoethanesulfonic acid (MES, a concentration of 0.05-0.1 M, pH = 5.0-6.0) was added to shake to mix well for 5 minutes, then placing the mixture on a magnetic grate for adsorption for 2 minutes, and discarding the supernatant;
3) repeating step 2) once;

### 1.2 Coupling of carboxyl magnetic beads with bispecific probes modified with primary amino group:

1) the centrifuge tube was removed from the magnetic grate to add 10 µL of bispecific probes modified with primary amino group (100 µM, 5'-end modified with primary amino group, a length of 20-70 bp), 100 µL of 1-(3-dimethylaminopropyl)-3-ethylcarbodiimide hydrochloride (10-20 mg/mL), and 1 mL of 2-morpholinoethanesulfonic acid respectively, with a volume ratio of 1:10:100;
2) the coupling mixture was placed in a shaking metal bath with constant temperature to incubate at room temperature (20-31°C) for 1-2 hours, then shaking at 4°C for 6-10 hours (to keep the magnetic beads suspended);

### 1.3 Blocking and storage:

1) After coupling was completed, the mixture was placed on a magnetic grate for adsorption for 2 minutes, then removing the supernatant, adding 1 mL of PBST solution (phosphate-tween buffer), pH = 7.0, containing 1% BSA (bovine serum albumin), shaking to mix well at 25°C for 1.5 hours to block the inactivated carboxyl groups on the surface of the magnetic beads;
2) the blocked magnetic beads were placed on a magnetic grate, then discarding the supernatant, adding 1 mL of 1% SDS washing solution (pH = 7.0-7.5) to remove any uncoupled probes, gently shaking to mix well, after standing at room temperature for 1 minutes, placing on a magnetic grate for adsorption for 2 minutes, and then discarding the supernatant;
3) repeating step 2) once;
4) 1 mL of TE buffer was added to the centrifuge tube to store at 2-8°C.

### Example 2: Extraction of Nucleic Acid from a Sample by Bispecific-probe-coupled Capture Magnetic Beads

### 2.1 Cell lysis:

1) 3 mL of cell lysis buffer (10-20 mmol/L EDTA, 100 mmol/L Tris, pH=7.5-8.0, 0.8% (W/V) SDS) and 2-4 mL of a sample (the supernatant of fecal sample, the precipitate of urine and ascites sample after centrifugation) were added to a 10-15 mL centrifuge tube;

### 2.2 Magnetic bead capture:

1) the mixture was heated at 95°C for 10-15 minutes, adding 50-100 µL of capture magnetic beads and 2 mL of 10%-40% polyethylene glycol (pH=6.0-7.0), then covering the tube to shake and mix well for 2 minutes, and standing at room temperature for 1 hour;
2) the centrifuge tube was placed on a magnetic grate for 5 minutes, then slowly aspirating and discarding the waste liquid;

### 2.3 Washing and storage:

1) 2 mL of washing solution 1 (2.0-3.0 M guanidine hydrochloride, isopropanol, pH = 6.5-7.5) was added to shake and mix well for 30 seconds, then placing the centrifuge tube in a magnetic separator for magnetic suction for 3 minutes, and completely aspirating and discarding the liquid;
2) repeating step 1) once;
3) 2 mL of washing solution 2 (70%-75% ethanol) was added to shake and mix well for 30 seconds, then placing the centrifuge tube in a magnetic separator for magnetic suction for 3 minutes, and completely aspirating and discarding the liquid;
4) repeating step 3) once;
5) the lid was open to let the centrifuge tube stand for 5 minutes at room temperature (20-30°C);
6) 50 µL of TE buffer (0.05-0.1 M Tris-HCl, 50 mM EDTA, PH=7.5-8.0) preheated to 50°C was added to shake and mix well for 30 seconds, eluting the magnetic beads on the wall of the centrifuge tube to the bottom of the tube, standing at room temperature for 5 minutes to elute, and then storing at 2-8°C.

### Example 3: Extraction of Target Nucleic Acid by Using Bispecific-probe-coupled Capture Magnetic Beads

In order to verify the difference between the bispecific-probe-coupled capture magnetic beads and the common capture beads, the capture probes were prepared for the methylated SDC2 gene and *Escherichia coli,* and human internal standard β-actin was added as the internal reference gene.
**Probe for capturing** methylated **SDC2 gene:**
   capture sequence: 5'-CGGCGTTTATTGGTTTTCGGAGTTGTTAATCGGCGTGTAA-3' (SEQ ID NO: 6).
**Bispecific probe for capturing** methylated **SDC2 gene:**
   capture sequence: 5'-CGGCGTTTATTGGTTTTCGGAGTTGIIIITCGGCGTGTAA-3' (SEQ ID NO: 1).
**Probe for capturing** *Escherichia coli*:
   capture sequence: 5'-AGTTTATCTGCAAGGTGATTCCTTAATTCCTCTCTTTCCT-3' (SEQ ID NO: 7).
**Bispecific probe for capturing** *Escherichia coli*:
   capture sequence: 5'-AGTTTATCTGCAAGGTGAIIIITTAATTCCTCTCTTTCCT-3' (SEQ ID NO: 5).
**Probe for capturing** methylated **β-actin gene:**
   capture sequence: 5'-TTGTAATTTTTAAGGGAGGAGTAGGTTTTATTGGTT-3' (SEQ ID NO: 8).
**Bispecific probe for capturing** methylated **β-actin gene:**
   capture sequence: 5'-TTGTAATTTTTAAGGGAGGAGIIIITTTTATTGGTT-3' (SEQ ID NO: 4).

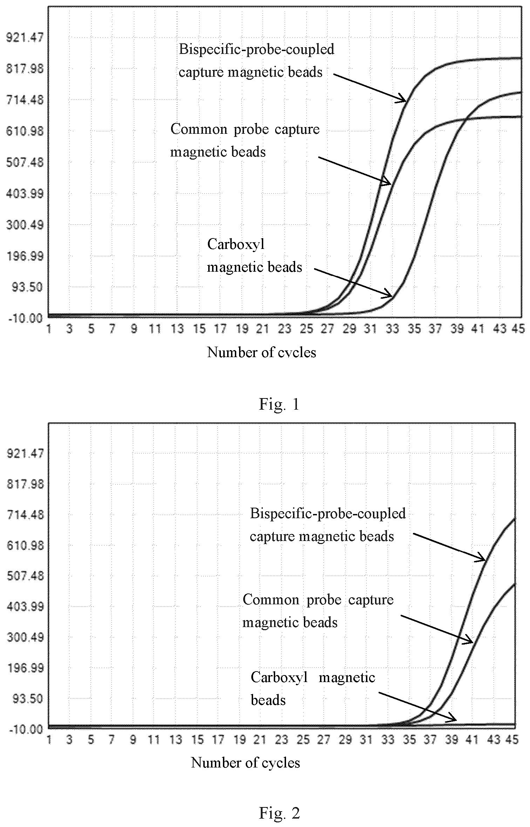

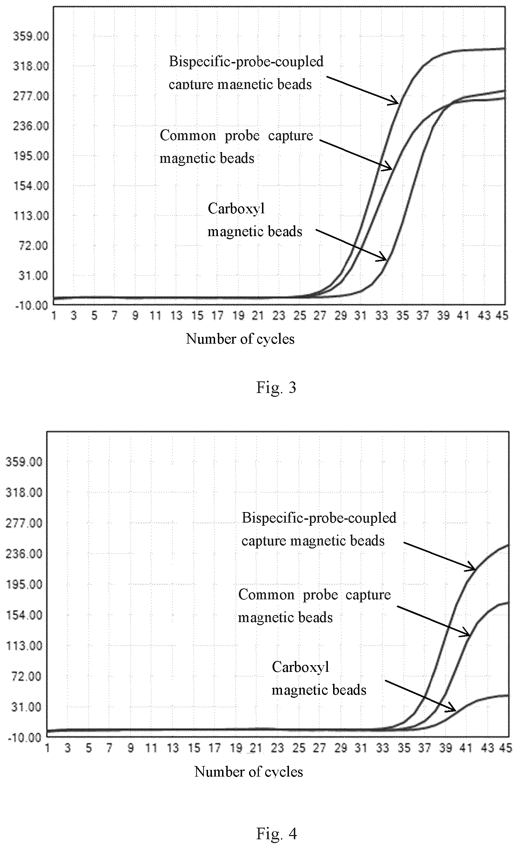

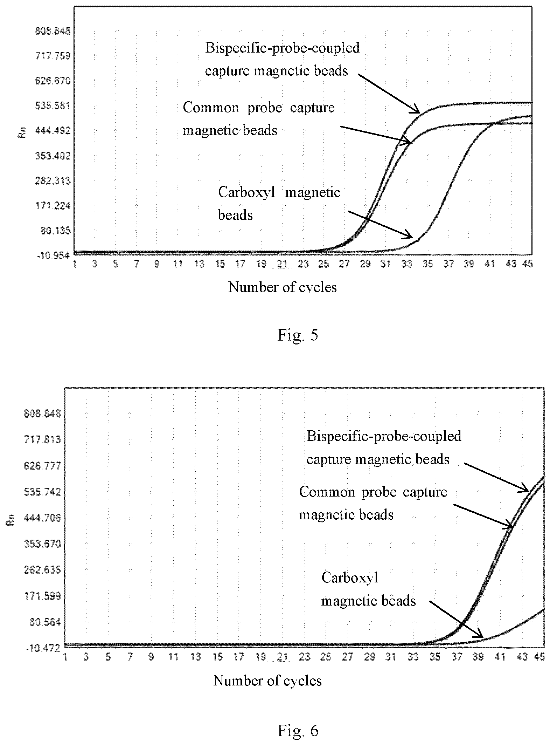

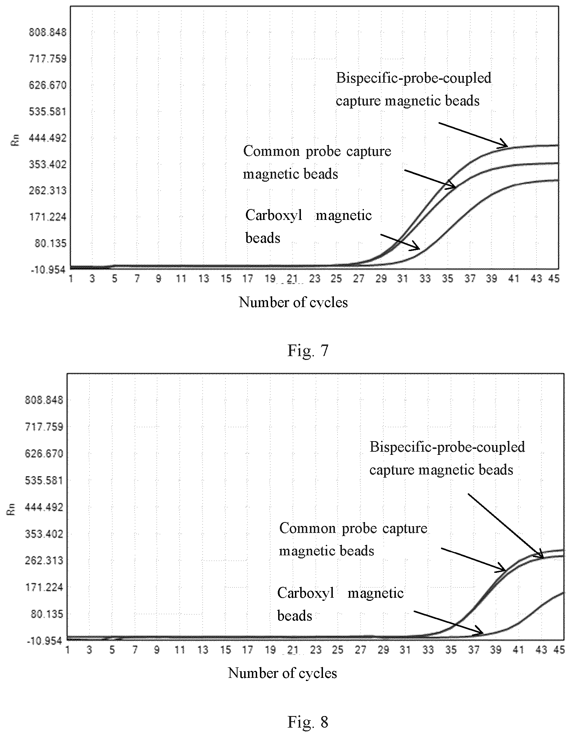

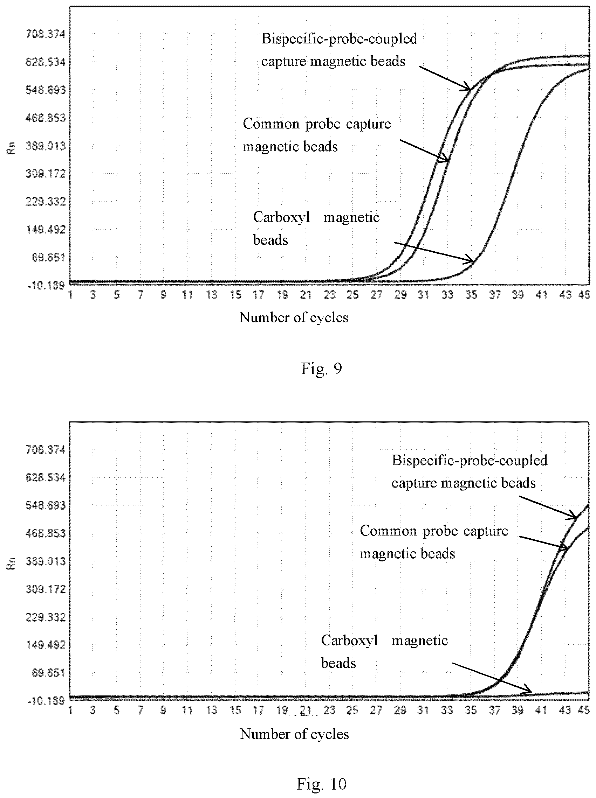

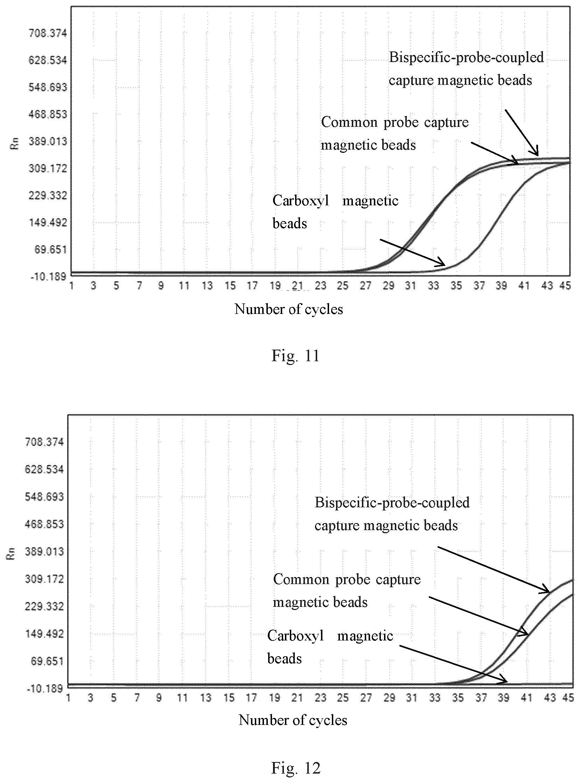

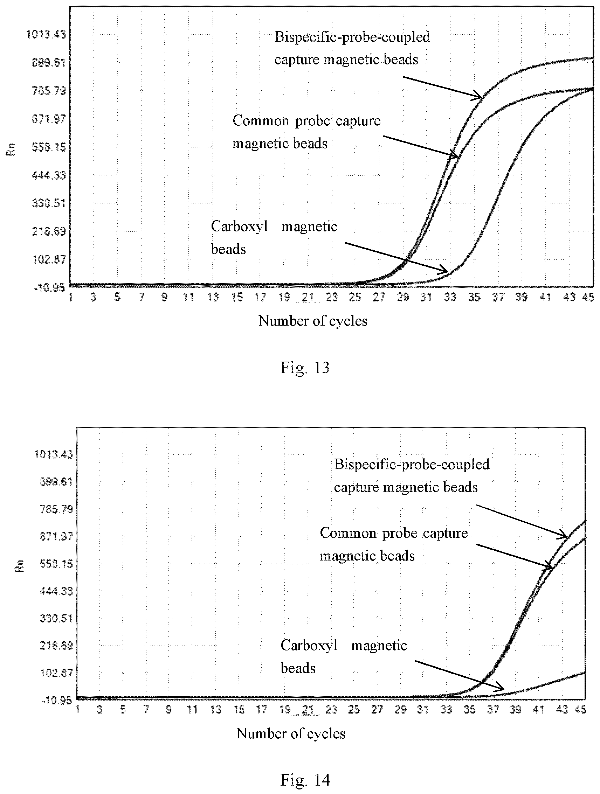

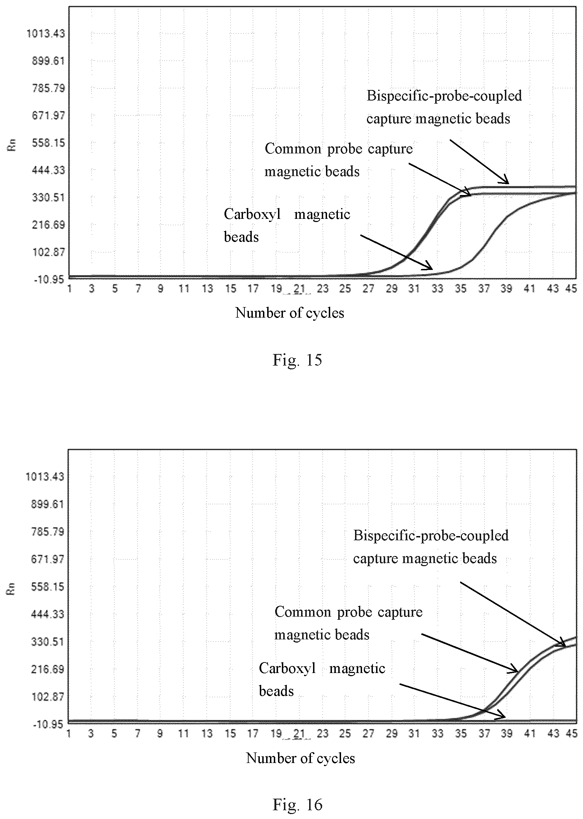

In order to verify the extraction effect of specific capture probes on methylation-positive samples, this experiment needs to use samples with high and low nucleic acid concentrations for testing. Since the amount of nucleic acid cannot be controlled during the extraction process, the test is carried out by adding artificially-quantified specific methylated fragments. The methylated fragments are standards of full methylated human genome. The methylated standards converted by sulfite are added to negative fecal supernatant (Figs. 1-4), plasma (Figs. 5-8), urine (Figs. 9-12) or hydrothorax and ascites (Figs. 13-16) to prepare simulated samples containing exogenous interferences. The high concentration of methylated fragments in the simulated samples is 100 ng/µL, and the low concentration of methylated fragments is 0.1 ng/µL. At the same time, unmethylated nucleic acid fragments are added as background signals, and the concentration of unmethylated fragment is 100 ng/µL.

### Specific capture:

The high concentration and low concentration simulated samples were extracted by using the bispecific-probe-coupled capture magnetic beads, common probe capture magnetic beads and carboxyl magnetic beads prepared in Example 1, respectively. The extraction steps were performed according to the operation of Example 2, and finally the nucleic acid was eluted with 50 µL of TE buffer.

### Sulfite conversion:

The nucleic acid extracted in Example 2 was subjected to sulfite conversion. The conversion kit was EZ DNA Methylation-lingthting Kit. The conversion steps were strictly carried out according to the operating instructions. Finally, the converted nucleic acid was eluted with 50 µL of TE buffer.

### PCR amplification detection:

The extracted nucleic acid was detected by fluorescent PCR, and primers and probe were designed based on the methylated SDC2 promoter region for testing and verification. To ensure the accuracy of the detection, a human internal standard β-actin was designed as an internal reference gene.
**Primers for detecting** methylated **SDC2 gene:**
   forward primer: 5'-CGTAGGAGTTTTGGTTTGTCG-3' (SEQ ID NO: 9),
   reverse primer: 5'-ACAATATAACTCCCAAATAAACCCG-3' (SEQ ID NO: 10).
**Probe for detecting** methylated **SDC2 gene:**
   fluorescent probe: 5'-TTCGGAGTTGTTAATCGGCGTG-3' (SEQ ID NO: 11).
**Primers for detecting** *Escherichia coli*:
   forward primer: 5'-TGTACAAGTCCACAAGGAAAGTAAAGAT-3' (SEQ ID NO: 12),
   reverse primer: 5'-TGTTTCGATGAGTTTATCTGCAAGGT-3' (SEQ ID NO: 13).
**Probe for detecting** *Escherichia coli*:
   fluorescent probe: 5'-TCTAACTAGGACCGCAGAGGAAAGAGAGGAATT-3' (SEQ ID NO: 14).
**Primers for detecting** methylated **β-actin gene:**
   forward primer: 5'-GTGTGTTGGGTGGTGGTTATTT-3' (SEQ ID NO: 15),
   reverse primer: 5'-CCAAAAAAAAAACTACTTATTCCAATTCAC-3' (SEQ ID NO: 16),
**Probe for detecting** methylated **β-actin gene:**
   fluorescent probe: 5'-TTAAGGGAGGAGTAGGTT-3' (SEQ ID NO: 17).

The PCR reaction system and amplification system are shown in Table 1 below:

**Table 1: PCR reaction system**

| Components | Volume (µL/component) |
|---|---|
| PCR buffer | Making up to 50µL |
| MgCl₂ (1mol/L) | 0.2 |
| dNTPs (100mM) | 1 |
| Target forward primer (50µM) | 0.2 |
| Target reverse primer (50µM) | 0.2 |
| Target fluorescent probe (50µM) | 0.1 |
| Internal standard forward primer (50µM) | 0.2 |
| Internal standard reverse primer (50µM) | 0.2 |
| Internal standard fluorescent probe (50µM) | 0.1 |
| DNA polymerase (5U) | 1 |
| Template | 10 |

**Table 2: The schedule of PCR fluorescence amplification**

| Temperature | Time | Number of cycles |
|---|---|---|
| 95°C | 5min | 1 |
| 95°C | 15s | 45 |
| 60°C | 30s (Collecting fluorescence) | |

The effects of different types of magnetic beads in extracting nucleic acids from different samples are shown in Tables 3-6 below.

**Table 3: Comparison of Ct values of amplification with different types of modified magnetic beads (feces samples)**

| Sample types | Repeating | Statistics of Ct values | | | | | |
|---|---|---|---|---|---|---|---|
| | | Carboxyl magnetic beads | | Common probe capture magnetic beads | | Bispecific-probe-coupled capture magnetic beads | |
| | | SDC2 | Internal standards | SDC2 | Internal standards | SDC2 | Internal standards |
| High concentration | 1 | 31.54 | 32.13 | 27.15 | 28.88 | 26.42 | 28.19 |
| | 2 | 31.61 | 32.24 | 27.44 | 29.03 | 26.58 | 28.07 |
| Low concentration | 1 | NoCt | 39.75 | 36.53 | 37.64 | 35.20 | 35.86 |
| | 2 | NoCt | No Ct | 36.86 | 37.15 | 35.47 | 36.12 |

**Table 4: Comparison of Ct values of amplification with different types of modified magnetic beads (plasma samples)**

| Sample types | Repeating | Statistics of Ct values | | | | | |
|---|---|---|---|---|---|---|---|
| | | Carboxyl magnetic beads | | Common probe capture magnetic beads | | Bispecific-probe-coupled capture magnetic beads | |
| | | SDC2 | Internal standards | SDC2 | Internal standards | SDC2 | Internal standards |
| High concentration | 1 | 33.84 | 32.59 | 26.76 | 27.36 | 26.40 | 27.51 |
| | 2 | 34.01 | 33.06 | 26.69 | 27.89 | 26.42 | 27.68 |
| Low concentration | 1 | 40.67 | 40.21 | 36.58 | 37.51 | 35.77 | 37.09 |
| | 2 | No Ct | 41.26 | 36.12 | 37.56 | 35.59 | 36.97 |

**Table 5: Comparison of Ct values of amplification with different types of modified magnetic beads (urine samples)**

| Sample types | Repeating | Statistics of Ct values | | | | | |
|---|---|---|---|---|---|---|---|
| | | Carboxyl magnetic beads | | Common probe capture magnetic beads | | Bispecific-probe-coupled capture magnetic beads | |
| | | SDC2 | Internal standards | SDC2 | Internal standards | SDC2 | Internal standards |
| High concentration | 1 | 34.64 | 35.71 | 27.59 | 27.74 | 26.83 | 27.69 |
| | 2 | 35.02 | 35.24 | 27.61 | 27.72 | 26.35 | 27.15 |
| Low concentration | 1 | No Ct | No Ct | 36.15 | 36.91 | 36.20 | 36.27 |
| | 2 | No Ct | No Ct | 36.27 | 36.49 | 36.36 | 36.42 |

**Table 6: Comparison of Ct values of amplification with different types of modified magnetic beads (ascites samples)**

| Sample types | Repeating | Statistics of Ct values | | | | | |
|---|---|---|---|---|---|---|---|
| | | Carboxyl magnetic beads | | Common probe capture magnetic beads | | Bispecific-probe-coupled capture magnetic beads | |
| | | SDC2 | Internal standards | SDC2 | Internal standards | SDC2 | Internal standards |
| High concentration | 1 | 35.31 | 36.48 | 27.68 | 27.90 | 28.11 | 27.74 |
| | 2 | 35.79 | 36.06 | 27.5 | 27.82 | 26.53 | 27.24 |
| Low concentration | 1 | 40.62 | No Ct | 37.16 | 38.06 | 36.41 | 38.04 |
| | 2 | No Ct | No Ct | 37.84 | 38.46 | 36.42 | 38.33 |

For fecal samples, the target nucleic acids captured by bispecific-probe-coupled capture magnetic beads were more than those captured by the common probe capture beads and carboxyl magnetic beads, and the Ct values were lower. At the same time, compared with carboxyl magnetic beads, bispecific-probe-coupled capture magnetic beads are less affected by inhibitors in complex samples and have higher capture efficiency, especially for the detection of low-concentration nucleic acid samples. The modified capture magnetic beads can effectively bind to the target fragment and eliminate the influence of interferences. Therefore, both bispecific-probe-coupled capture magnetic beads and common capture magnetic beads can effectively distinguish target fragments from interferences. The extraction effect of carboxyl magnetic beads is the worst. Due to the interference of unmethylated nucleic acid fragments, carboxyl magnetic beads cannot effectively and selectively bind to target fragments, and the extraction concentration of target fragments in feces, urine, plasma, or ascites samples is low.

In urine, plasma and ascites samples, there are fewer exogenous interferences and the sample environment is significantly purer than that of fecal samples. Therefore, there is no significant difference in the capture efficiency between bispecific-probe-coupled capture magnetic beads and common probe capture magnetic beads, and both can capture specific target fragments.

As for the capture of *E. coli,* the results are shown in Table 7 below.

**Table 7: Comparison of Ct values of amplification with different types of modified magnetic beads (feces samples)**

| Sample types | Repeating | Statistics of Ct values | | | | | |
|---|---|---|---|---|---|---|---|
| | | Carboxyl magnetic beads | | Common probe capture magnetic beads | | Bispecific-probe-coupled capture magnetic beads | |
| | | *E. coli* | Internal standards | *E. coli* | Internal standards | *E. coli* | Internal standards |
| Fecal samples | 1 | 36.94 | 32.18 | 34.08 | 31.72 | 31.93 | 30.46 |
| | 2 | 36.32 | 31.61 | 34.81 | 31.38 | 32.11 | 30.60 |

It can be seen from the table that the bispecific-probe-coupled capture magnetic beads have a better capture effect on *Escherichia coli.*

### Example 4: Extraction of Target Nucleic Acid by Combining Bispecific-probe-coupled Capture Magnetic Beads with PEG

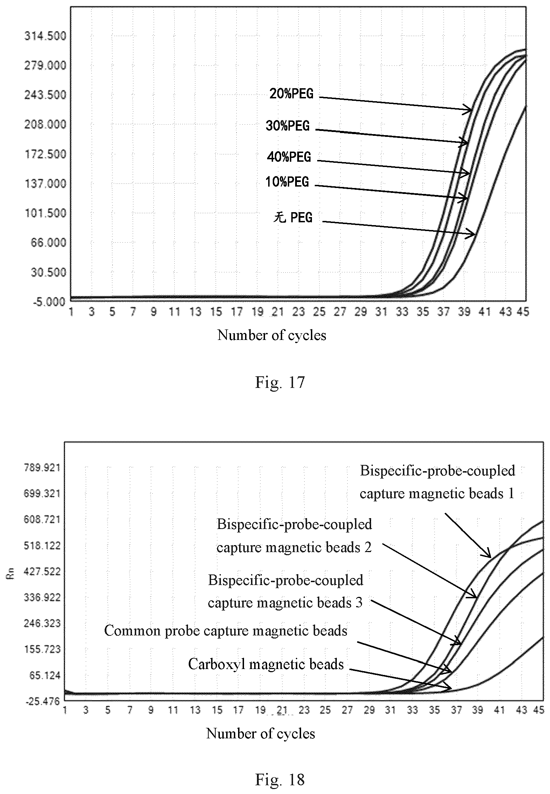

During the extraction process of bispecific-probe-coupled capture magnetic beads, we found that adding a certain concentration of polyethylene glycol can increase the complementary binding rate of capture sequences. A comparative test was conducted by adding different concentrations of polyethylene glycol during the extraction process, and the results are shown in Fig. 17. As can be seen from the Fig. 17, the extraction effect of adding 20% or 30% PEG is better than that of adding other concentrations of PEG.

### Example 5: Effect of Introducing Hypoxanthine at Different Positions of the Capture Probe on the Capture Efficiency

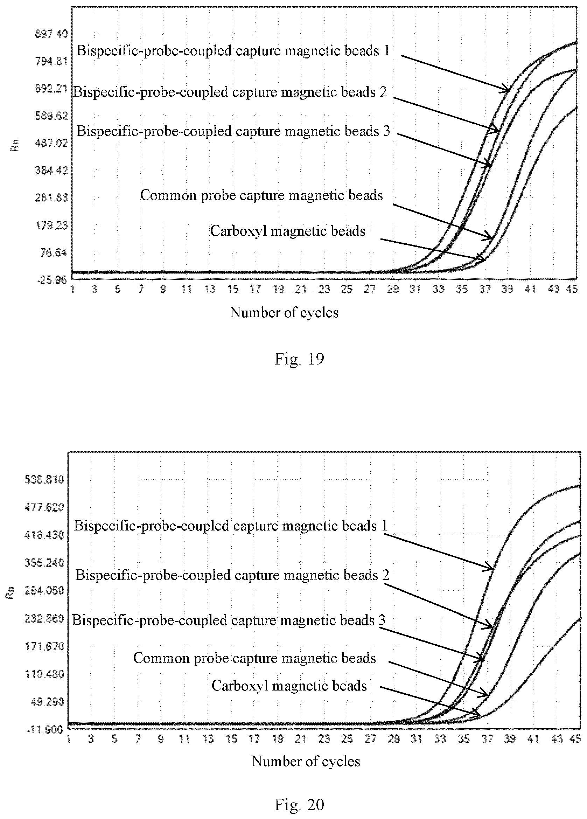

In order to verify the effect of introducing hypoxanthine at different positions of the bispecific-probe-coupled capture magnetic beads according to the present application on the capture efficiency, the capture probes of methylated target genes SDC2, NDRG4 and Septin9 were used, and hypoxanthine was introduced at the 5' end, 3' end and middle region of the capture sequence, so as to compare the nucleic acid extraction effect of the capture magnetic beads. The results are shown in Figs. 18-20. It can be seen from the figures that the introduction of hypoxanthine in a region 15-25 bp away from the 5' end of the bispecific probe can greatly improve the sensitivity of the capture probe.
**Probe for capturing** methylated **SDC2 gene:**
   capture sequence: 5'-CGGCGTTTATTGGTTTTCGGAGTTGTTAATCGGCGTGTAA-3' (SEQ ID NO: 6).
**Bispecific probe 1 for capturing** methylated **SDC2 gene:**
   capture sequence: 5'-CGGCGTTTATTGGTTTTCGGAGTTGIIIITCGGCGTGTAA-3' (SEQ ID NO: 1).
**Bispecific probe 2 for capturing** methylated **SDC2 gene:**
   capture sequence: 5'-CIIIITTTATTGGTTTTCGGAGTTGTTAATCGGCGTGTAA-3' (SEQ ID NO: 18).
**Bispecific Probe 3 for capturing** methylated **SDC2 gene:**
   Capture sequence: 5'-CGGCGTTTATTGGTTTTCGGAGTTGTTAATCGGCGIIIIA-3' (SEQ ID NO: 19).
**Probe for capturing** methylated **NDRG4** gene :
   Capture sequence: 5'-TTTATCGGGTATTTTAGTCGCGTAGAAGGCGGAAGTTACG-3' (SEQ ID NO: 20).
**Bispecific probe 1 for capturing** methylated **NDRG4 gene:**
   capture sequence: 5'-TTTATCGGGTATTTTAGTCIIIIAGAAGGCGGAAGTTACG-3' (SEQ ID NO: 2).
**Bispecific probe 2 for capturing** methylated **NDRG4 gene:**
   capture sequence: 5'-TTIIIIGGGTATTTTAGTCGCGTAGAAGGCGGAAGTTACG-3' (SEQ ID NO: 21).
**Bispecific probe 3 for capturing** methylated **NDRG4 gene:**
   capture sequence: 5'-TTTATCGGGTATTTTAGTCGCGTAGAAGGCGGAAIIIICG-3' (SEQ ID NO: 22).
**Probes for capturing** methylated **Septin9 gene:**
   capture sequence: 5'-TTAGTTATTATGTCGGATTTCGCGGTTAACGCGTAGTTGG-3' (SEQ ID NO: 23).
**Bispecific probe 1 for capturing** methylated **Septin9 gene:**
   capture sequence: 5'-TTAGTTATTATGTCGGATTTIIIIGTTAACGCGTAGTTGG-3' (SEQ ID NO: 3).
**Bispecific probe 2 for capturing** methylated **Septin9 gene:**
   capture sequence: 5'-TTIIIIATTATGTCGGATTTCGCGGTTAACGCGTAGTTGG-3' (SEQ ID NO: 24).
**Bispecific probe 3 for capturing** methylated **Septin9 gene:**
   capture sequence: 5'-TTAGTTATTATGTCGGATTTCGCGGTTAACGCGTIIIIGG-3' (SEQ ID NO: 25).

### Comparative Example 1: Capture Accuracy of Different Capture Probes

In order to verify the capture accuracy of the bispecific-probe-coupled capture magnetic bead according to the present application in fecal samples, the methylated target gene Septin9 of colorectal cancer was used as the capture target fragment, and 1-3 mismatched bases were introduced into the synthesized methylated fragment. The fragments introduced with mismatched bases were added to the negative fecal supernatant to prepare a simulated sample. The concentration of mismatched methylated fragments in the simulated sample was 100 ng/µL. At the same time, bispecific magnetic beads, commmon magnetic beads and carboxyl magnetic beads were used to compare the nucleic acid extraction effect.
**Methylated sequence of Septin9 gene introduced with one mismatched base:**
   Mismatched sequence: 5'-TTAGTTATTATGTTGGATTTCGCGGTTAACGCGTAGTTGG-3' (SEQ ID NO: 26).
**Methylated sequence of Septin9 gene introduced with three mismatched bases:**
   mismatched sequence: 5'-TTAGTTATTATGTTGGATTTCGCGGTTAATGTGTAGTTGG-3' (SEQ ID NO: 27).

**Table 8: Comparison of Ct values of amplification with different capture beads**

| Septin9 | Repeating | Statistics of Ct values | | |
|---|---|---|---|---|
| | | Bispecific capture probes | Common capture probes | Carboxyl magnetic beads |
| 1 mismatched base | 1 | No Ct | 34.18 | 26.32 |
| | 2 | No Ct | 35.62 | 26.47 |
| 3 mismatched bases | 1 | No Ct | 37.96 | 26.09 |
| | 2 | No Ct | 38.16 | 26.28 |

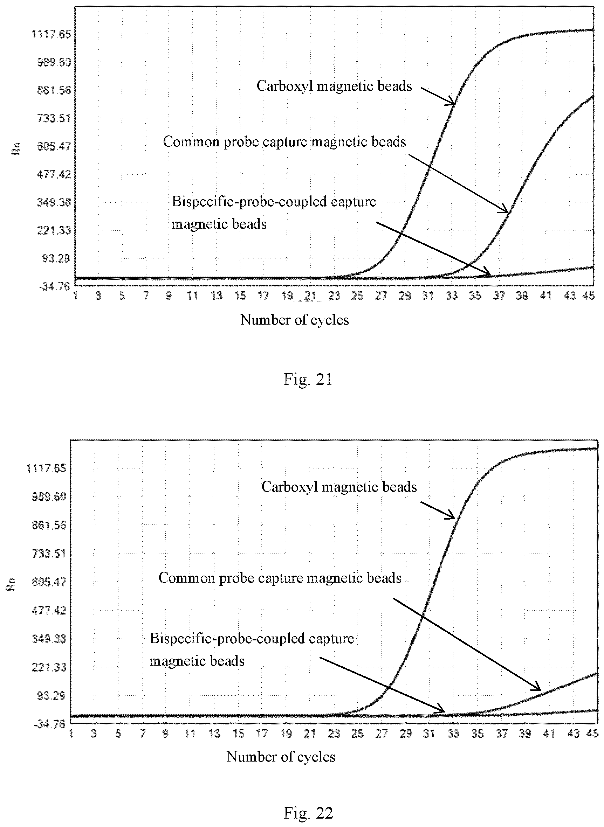

It can be seen from Figs. 21-22 and Table 8 that carboxyl magnetic beads cannot recognize the accurate nucleic acid sequences. They can bind to any nucleic acids as long as they are present, so the nucleic acid concentration in the test result is the highest. When there is only 1-3 mismatched bases difference in the sequence, the capture probes of common capture magnetic beads still bind to a small amount of mismatched sequence making the test result positive. Due to the introduction of hypoxanthine, the capture sequence specificity of the bispecific capture magnetic beads is enhanced; for the presence of mismatched bases in the sequence that cannot bind to the magnetic beads normally, the test result is negative, thus its capture accuracy is the highest. Therefore, it may be seen that the capture accuracy of the bispecific probe of the present application is much higher than that of the common probe and the carboxyl magnetic beads, especially when there is only one mismatched base, the capture accuracy is significantly improved.

## Claims

1. A method for preparing a bispecific-probe-coupled capture magnetic bead, **characterized in that** the method comprises the following steps:
coupling a capture magnetic bead with a bispecific probe,
wherein hypoxanthine is introduced into a position in the 15-25 bp region at the 5' end of the bispecific probe, and the bispecific probe has a length of 20-70 bp.

2. The method according to claim 1, **characterized in that** a target of the bispecific probe is SDC2 gene, NDRG4 gene, Septin9 gene, β-actin gene, and/or *Escherichia coli.*

3. The method according to claim 2, **characterized in that** a sequence of the bispecific probe is one or more selected from the sequences represented by SEQ ID NOs: 1-5.

4. The method according to any one of claims 1-3, **characterized in that** the capture magnetic bead is a carboxyl magnetic bead, and 5' end of the bispecific probe is modified with a primary amino group.

5. The method according to claim 4, **characterized in that** coupling the bispecific probe with the carboxyl magnetic bead comprises the following steps:
S1. placing the magnetic beads on a magnetic grate for adsorption;
S2. adding 2-morpholinoethanesulfonic acid to shake and mix well, then placing the mixture on a magnetic grate for adsorption and activation; and
S3. incubating the bispecific probe with the activated carboxyl magnetic bead from step S2, 2-morpholinoethanesulfonic acid, and 1-(3-dimethylaminopropyl)-3-ethylcarbodiimide hydrochloride.

6. A bispecific-probe-coupled capture magnetic bead, **characterized in that** the bead is prepared by the method according to any one of claims 1-5.

7. A kit for extracting nucleic acids, **characterized by** comprising the bispecific-probe-coupled capture magnetic bead according to claim 6.

8. The kit according to claim 7, **characterized in that** the kit further comprises polyethylene glycol.

9. The kit according to claim 7, **characterized in that** the concentration of the polyethylene glycol is 20-30%.

10. Use of the bispecific-probe-coupled capture magnetic bead according to claim 6 in the preparation of a kit for extracting nucleic acids.
